# EUROPÄISCHE PATENTSCHRIFT

(11) **EP 1 888 740 B1**
(45) Veröffentlichungstag und Bekanntmachung des Hinweises auf die Patenterteilung: **21.10.2009**
(21) Anmeldenummer: 06753237.4
(22) Anmeldetag: 01.06.2006
(51) Int. Cl.: C12N 1/20, C12P 17/14, C12P 17/16

(54) **MIKROORGANISMEN BURKHOLDERIA RHIZOXINA, NEUE ENDOSYMBIONTEN AUS RHIZOPUS SP. UND VERFAHREN ZUR HERSTELLUNG VON RHIZOXIN UND / ODER RHIZOXIN-DERIVATEN UNTER VERWENDUNG DIESER MIKROORGANISMEN**
BURKHOLDERIA RHIZOXINA MICRO-ORGANISMS, NOVEL ENDOSYMBIONTS OF RHIZOPUS SP. AND METHOD FOR PRODUCING RHIZOXIN AND/OR RHIZOXIN DERIVATIVES USING SAID MICRO-ORGANISMS
MICRO-ORGANISMES BURKHOLDERIA RHIZOXINA, NOUVEAUX ENDOSYMBIOTES DE RHIZOPUS SP., ET PROCEDE DE PRODUCTION DE RHIZOXINE ET/OU DE DERIVES DE RHIZOXINE AU MOYEN DE CES MICRO-ORGANISMES

(30) Priorität: 06.06.2005 DE 102005026417
(43) Veröffentlichungstag der Anmeldung: 20.02.2008
(73) Patentinhaber: Leibniz-Institut für Naturstoff-Forschung und Infektionsbiologie e.V. -Hans-Knöll-Institut- (HKI), 07745 Jena (DE)
(72) Erfinder: HERTWECK, Christian, 04105 Leipzig (DE); PARTIDA-MARTINEZ, Laila, P., 07743 Jena (DE)
(74) Vertreter: Donath, Dirk
(86) Internationale Anmeldenummer: PCT/DE2006/000962
(87) Internationale Veröffentlichungsnummer: WO 2006/131098

(56) Entgegenhaltungen:
- EP-A2- 0 132 772
- EP-A2- 0 145 177
- PARTIDA-MARTINEZ LAILA P ET AL: "Pathogenic fungus harbours endosymbiotic bacteria for toxin production" NATURE (LONDON), Bd. 437, Nr. 7060, Oktober 2005 (2005-10), Seiten 884-888, XP002408671 ISSN: 0028-0836
- JENNESSEN JENNIFER ET AL: "Secondary metabolite and mycotoxin production by the Rhizopus microsporus group" JOURNAL OF AGRICULTURAL AND FOOD CHEMISTRY, Bd. 53, Nr. 5, März 2005 (2005-03), Seiten 1833-1840,1823, XP002408672 ISSN: 0021-8561 in der Anmeldung erwähnt
- IWASAKI S ET AL: "MACRO CYCLIC LACTONE ANTIBIOTICS 7. STRUCTURE OF A PHYTO TOXIN RHIZOXIN PRODUCED BY RHIZOPUS-CHINENSIS" JOURNAL OF ANTIBIOTICS (TOKYO), Bd. 37, Nr. 4, 1984, Seiten 354-362, XP009075351 ISSN: 0021-8820

## Beschreibung

Mikroorganismen *Burkholderia rhizoxina,* neue Endosymbionten aus *Rhizopus* sp. und Verfahren zur Herstellung von Rhizoxin und / oder Rhizoxin-Derivaten unter Verwendung dieser Mikroorganismen

Erfindung betrifft Mikroorganismen *Burkholderia rhizoxina,* neue Endosymbionten aus *Rhizopus* sp. und Verfahren zur Herstellung von Rhizoxin und / oder Rhizoxin-Derivaten unter Verwendung dieser Mikroorganismen.
Rhizoxin [(1) in Tabelle 1] ist ein Polyketidmetabolit mit antifungaler und zytotoxischer Wirkung, der aus verschiedenen Pilzen der Gattung *Rhizopus* (z.B. *Rhizopus microsporus*) isoliert wurde (J. Jennessen et al. 2005, J. Agric. Food Chem. 53: 1833-18409).
Die zytotoxische Wirkung des Rhizoxin kommt durch eine sehr effektive Bindung an β-Tubulin zustande, was die Hemmung der Mitose zu Folge hat (Y. Koga-Ban et al. 1995, DNA Res. 2: 21-26; M. Takahashi et al. 1987, Biochim. Biophys. Acta 926: 215-223).
Aufgrund dieser antimitotischen Aktivität, die in den meisten eukaryotischen Zellen beobachtet wird, insbesondere in verschiedenen humanen Tumorzelllinien, hat Rhizoxin großes Interesse als potentieller Antitumorwirkstoff geweckt (T. Tsuruo et al. 1986, Cancer Res. 46: 381-385; A. Jordan et al. 1998, Med. Res. Rev. 18: 259-296).
Ähnlich starke antifungale und antitumorale Wirkungen wurden auch für die Rhizoxin-Derivate [(29 bis (10) in Tabelle 1] beobachtet (S. Kiyoto et al. 1986, J. Antibiot. 39: 762-772; S. Iwasaki et al. 1986, J. Antibiot. 39: 424-429; S. Iwasaki et al., 1986, Chem. Pharm. Bull. 34: 1387-1390).
Die Produktion von Rhizoxin und seinen Derivaten durch Fermentation von *Rhizopus*-Stämmen und die Aufreinigung der zytotoxischen Verbindungen ist jedoch aufwendig und die Ausbeute an Rhizoxin oder Derivaten von Rhizoxin ist gering.

Aufgabe der Erfindung ist es, neue Mikroorganismen bereitzustellen, die Rhizoxin oder Derivaten von Rhizoxin synthetisieren und ein Verfahren zur Herstellung von Rhizoxin oder Derivaten von Rhizoxin anzugeben, das die Nachteile des Standes der Technik vermeidet und Rhizoxin und / oder seine Derivate aufwandgeringer und mit einer höheren Ausbeute bereitstellt.

Unter dem Ausdruck "Derivat" im Sinne der vorliegenden Erfindung werden Varianten verstanden, die die gleichen funktionellen Eigenschaften wie Rhizoxin besitzen. Die Derivate können auch eingeschränkte funktionelle Eigenschaften besitzen.

Überraschender weise wurde gefunden, dass nicht die *Rhizopus* sp.-Stämme die Produzenten von Rhizoxin sind, sondern bakterielle Endosymbionten *Burkholderia rhizoxina,* die eng mit Spezies der Gattung *Burkholderia* verwandt sind, diese Naturstoffe produzieren und dass diese bakteriellen Endosymbionten aus *Rhizopus* sp. dazu geeignet sind, die Herstellung von Rhizoxin und / oder seinen Derivaten aufwandgeringer und mit einer höheren Ausbeute in Flüssigkultur zu ermöglichen.

Die Isolation der bakteriellen Endosymbionten erfolgt erfindungsgemäß aus Rhizoxin-Bildnern, wie z.B. *Rhizopus microsporus* van Tieghem var. *chinensis* (ATCC 62417), *Rhizopus* sp. F-1360 (ATCC 20577), *Rhizopus microsporus var. microsporus* (Stämme CBS 699.68 und CBS 308.87), indem man die *Rhizopus*-Stämme in flüssigen Medium (1 % Mais-Stärke, 0.5 % Glycerol, 1 % Glutenmehl, 1 % Hefe, 1 % Cornsteep, und 1 % CaCO₃ - pH 6.5) oder Malzextrakt-Pepton-Medium bei 30 °C und Schütteln bei 120-200 rpm kultiviert, das Mycel einer wachsenden *Rhizopus*-Kultur (bspw. 20 ml) anschließend in ein Gefäß (bspw. 50 ml-Gefäß) überführt, mechanisch durch Pipettieren zerteilt und mechanisch aufschließt.
Die aufgeschlossenen Zellen waren einer Gradientenzentrifugation unterworfen (bspw. 500 rpm für 5 min, 4000 rpm für 20 min).
Nach jedem Zentrifugationsschritt werden Aliquots des Überstands auf Agarplatten (5.0 g Pepton, 3.0 g Fleischextrakt, 15.0 g Agar, 1000 ml Wasser) ausgestrichen und bei 30 °C inkubiert. Die vereinzelten Kolonien des Endosymbionten sind nach der Kultivierung als Reinkulturen von den Platten isolierbar.

Es ist bspw. der Endosymbionten-Stamm DSM 17360 (*Burkholderia rhizoxina*) isolierbar.
Der Stamm *Burkholderia rhizoxina* (DSM 17360) wurde in der Deutschen Sammlung für Mikroorganismen und Zellkulturen - GmbH (DSMZ) in Braunschweig, Mascheroder Wege 1, unter der Bezeichnung hinterlegt.

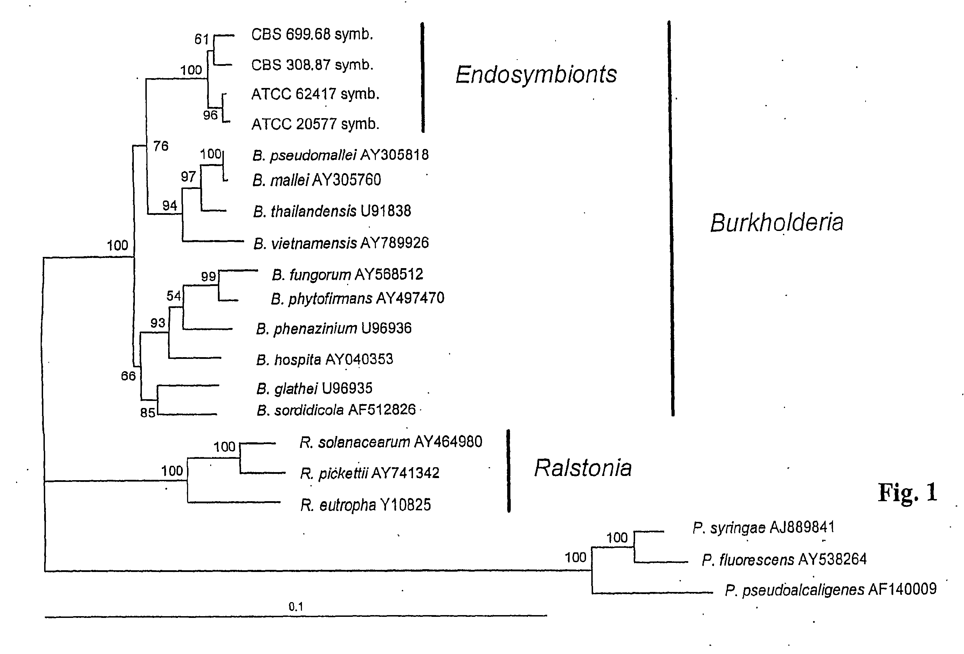

Der Endosymbionten-Stamm DSM 17360 unterscheidet sich von den bisher bekannten Stämmen anhand seiner 16S r-RNA-Sequenz, wie in der phylogenetischen Veranschaulichung in Fig. 1 dargestellt.

Erfindungsgemäß können die auf diese Weise erhaltenen bakteriellen Kolonien (*Burkholderia rhizoxina*-Stämme) für die Herstellung von Rhizoxin und Rhizoxin-Derivaten wie folgt eingesetzt werden:
Die Fermentation der Bakterien-Stämme erfolgt als Submerskultur in flüssigen Medium (1 % Maisstärke, 0.5 % Glycerol, 1 % Glutenmehl, 1 % Hefe, 1 % Cornsteep und 1 % CaCO₃- pH 6.5) bei 30 °C oder 5.0 g Pepton und 3.0 g Fleischextrakt in 1000 ml Wasser bei 30 °C.

Das Rhizoxin (1) bzw. die Rhizoxin-Derivate (2 bis 10) gemäß Tabelle 1 sind aus dem Kulturüberstand gewinnbar, indem mit organischen Lösungsmitten, z.B. Ethylacetat, extrahiert wird. Alternativ können bekannte Verfahren zur Festphasenextraktion eingesetzt werden.

**Tabelle 1**

| **Strukturformel** | **Name** | **Masse** | **Summenformel** |
|---|---|---|---|
| 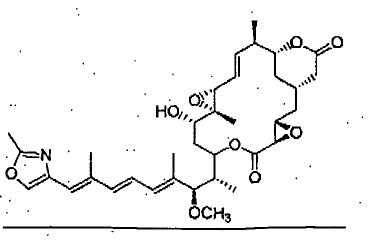 | Rhizoxin (→) | 625,8 | G35H47NO9 |
| 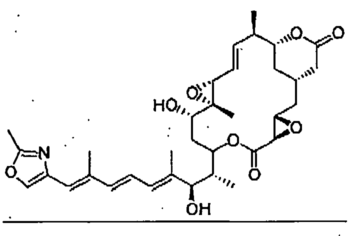 | **2** | 611,7 | C34H45NO9 |
| 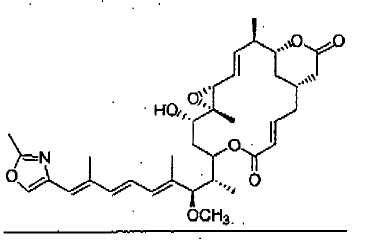 | **3** | 609,8 | C35H47NO8 |
| 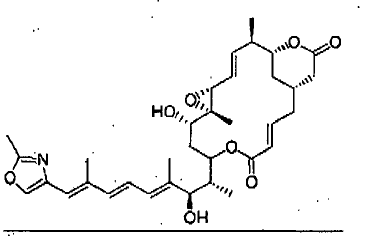 | **4** | 595,7 | C34H45NO8 |
| 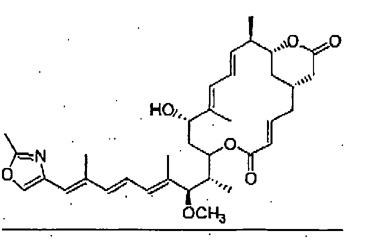 | **5** | 593,8 | C35H47NO7 |
| 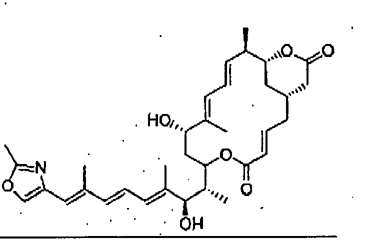 | **6** | 579,7 | C34H45NO7 |
| 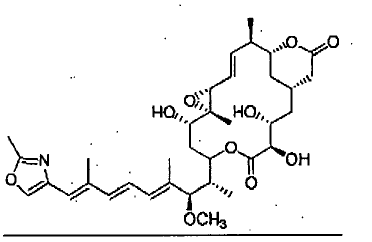 | **7** | 643,8 | C35H494NO10 |
| 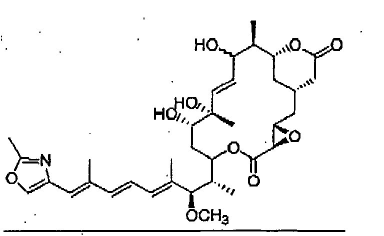 | **8** | 643,8 | C35H49NO10 |
| 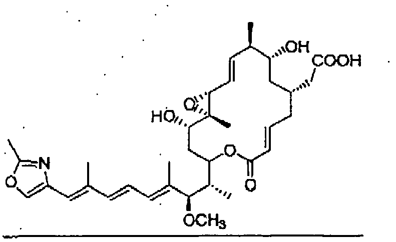 | **9** | 627,8 | C35H49NO9 |
| 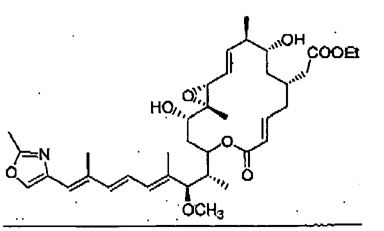 | **10** | 655,8 | C37H53NO9 |

Die Ausbeute an Rhizoxin bzw. die Rhizoxin-Derivate liegt dabei in einem Bereich von 10 mg/l bis über 200 mg/l Kulturüberstand.

## Patentansprüche

1. *Burkholderia rhizoxina* DSM 17360.

2. Verwendung von *Burkholderia rhizoxina* gemäß Anspruch 1, zur Herstellung von Rhizoxin (1) 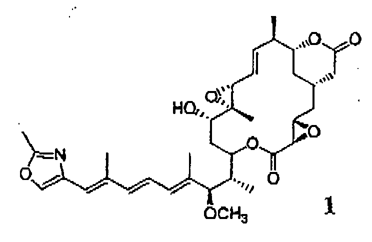 und / oder Derivaten des Rhizoxins (2) bis (10)

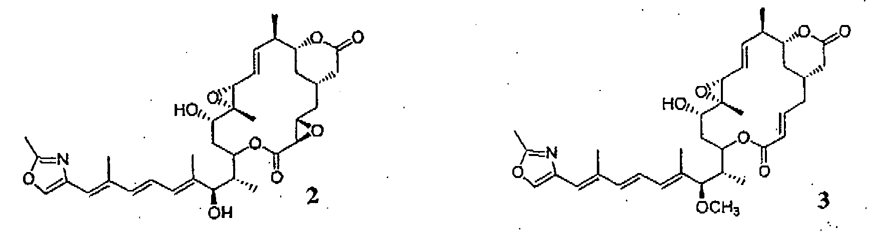

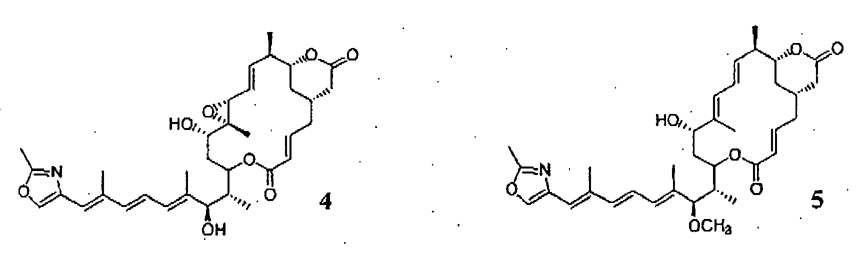

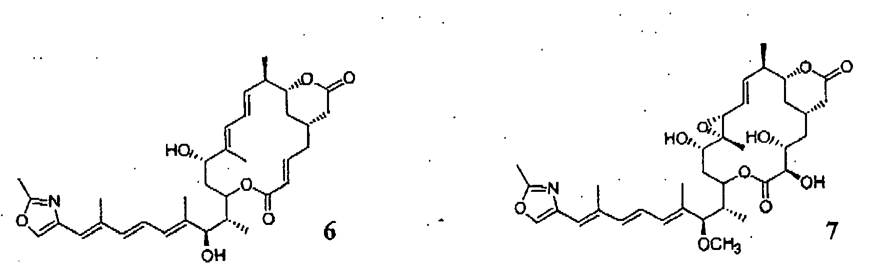

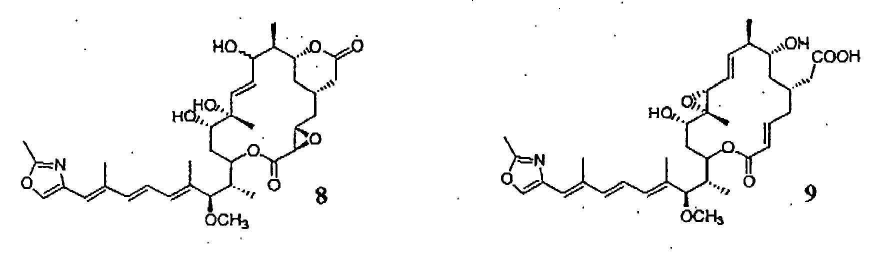

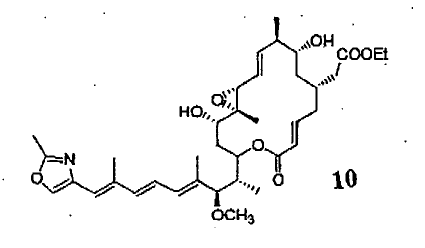

3. Verfahren zur Herstellung von Rhizoxin (1) 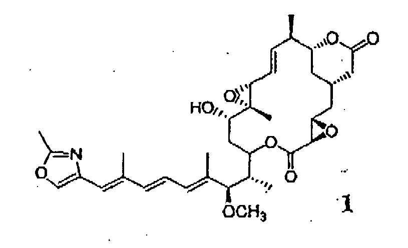 und / oder Derivaten des Rhizoxins (2) bis (10) 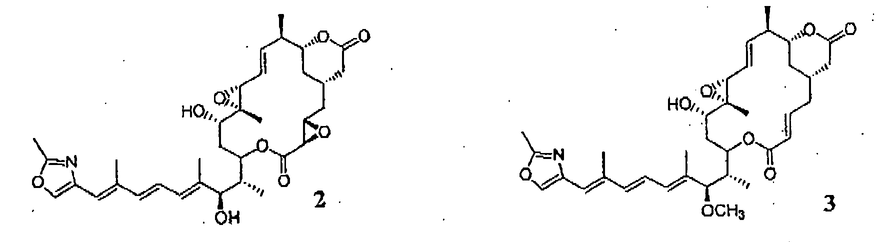 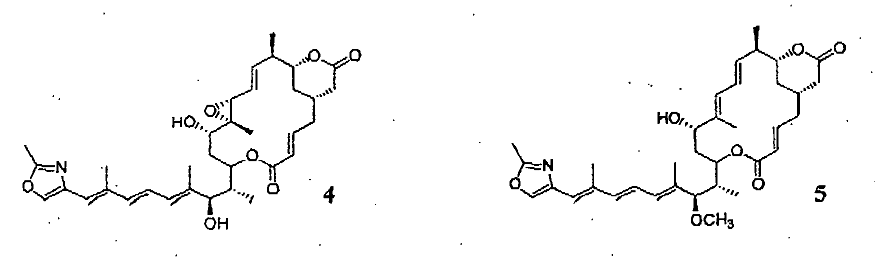 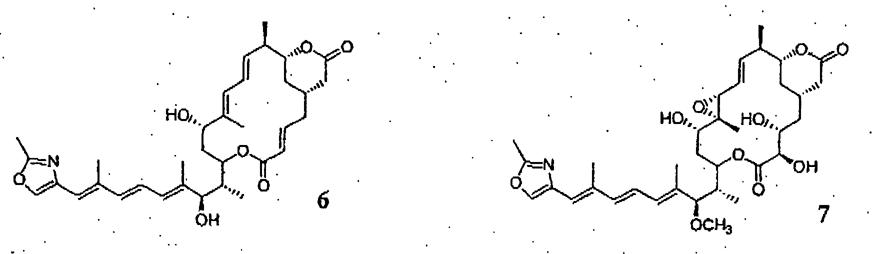 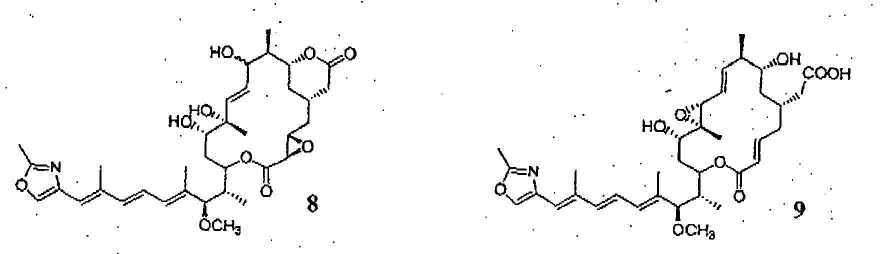 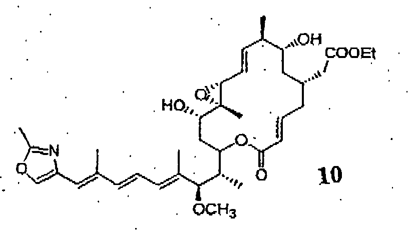 bei dem ein bakterieller Symbiont aus *Rhizopus* sp. in Form eines *Burkholderia Rhizoxina*-Stammes in Submerskultur kultiviert wird, bis sich Rhizoxin (1) und / oder Derivaten des Rhizoxins (2) bis (10) im Kulturüberstand ansammeln und aus dem Kulturüberstand isoliert werden.

4. Verfahren zur Herstellung von Rhizoxin (1) und/oder Derivaten des Rhizoxin (2) bis (10) nach Anspruch 3, **dadurch gekennzeichnet, dass** der *Burkholderia Rhizoxina*-Stamm DSM 17360 ist.

5. Verfahren zur Herstellung von Rhizoxin (1) und / oder Derivaten des Rhizoxins (2) bis (10) nach Anspruch 3, **dadurch** gekenntzeichnet, dass das Medium 1 % Mais-Stärke, 0.5 % Glycerol, 1 % Glutenmehl, 1 % Hefe, 1 % Cornsteep, und 1 % CaCO₃ enthält.

6. Verfahren zur Herstellung von Rhizoxin (1) und / oder Derivaten des Rhizoxins (2) bis (10) nach Anspruch 3, **dadurch gekennzeichnet, dass** das Medium ein Malzextrakt-Pepton-Medium ist.

7. Verfahren zur Herstellung von Rhizoxin (1) und / oder Derivaten des Rhizoxins (2) bis (10) nach Anspruch 5 oder 6, **dadurch gekennzeichnet, dass** der pH-Wert 6.5 beträgt.

8. Verfahren zur Herstellung von Rhizoxin (1) und / oder Derivaten des Rhizoxins (2) bis (10) nach Anspruch 5 oder 6, **dadurch gekennzeichnet, dass** die Fermentationstemperatur 30 °C beträgt.

9. Verfahren zur Herstellung von Rhizoxin (1) und / oder Derivaten des Rhizoxins (2) bis (10) nach Anspruch 5 oder 6, **dadurch gekennzeichnet, dass** bei 120-200 rpm kultiviert wird.

10. Verfahren zur Herstellung von Rhizoxin (1) und / oder Derivaten des Rhizoxins (2) bis (10) nach Anspruch 4, **dadurch gekennzeichnet, dass** das Rhizoxin (1) und / oder seine Derivaten (2) bis (10) aus dem Kulturüberstand durch Extraktion mit organischen Lösemitteln oder Adsorberharzen isoliert werden.

## Claims

1. *Burkholderia rhizoxina* DSM 17360.

2. Use of *Burkholderia rhizoxina* according to claim 1, for the production of rhizoxin (1) 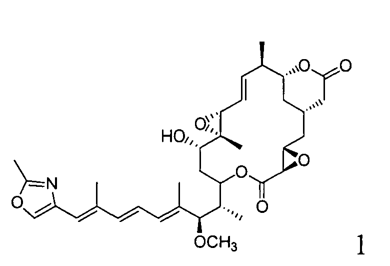 and / or rhizoxin derivatives (2) to (10)

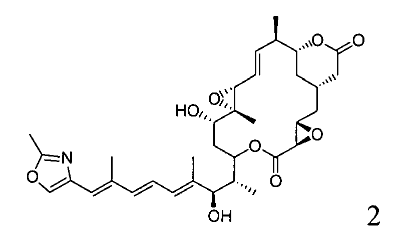

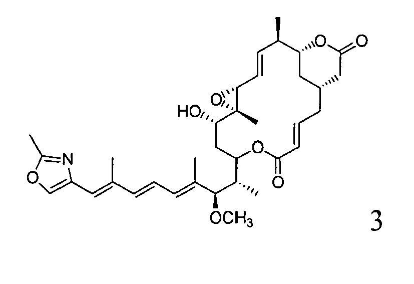

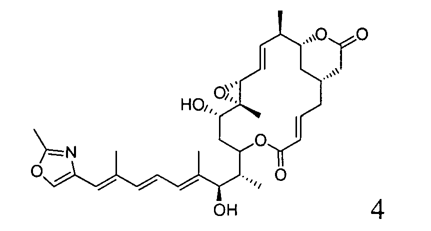

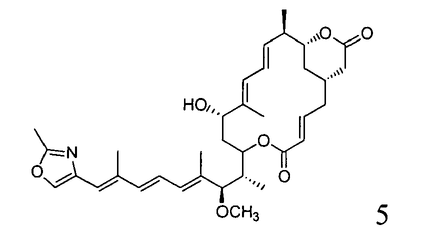

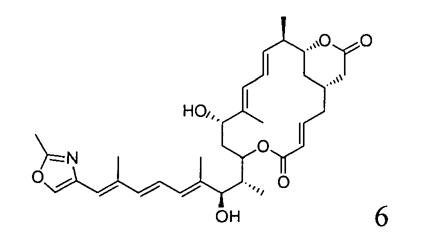

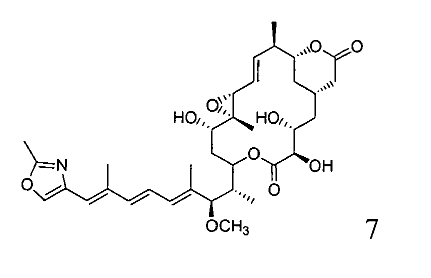

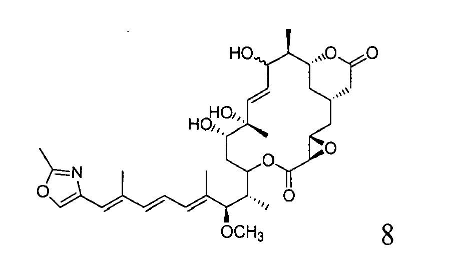

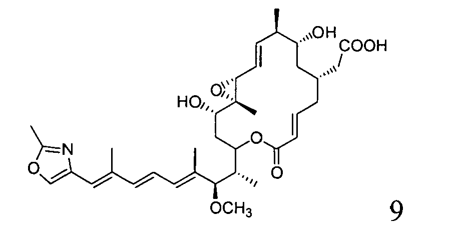

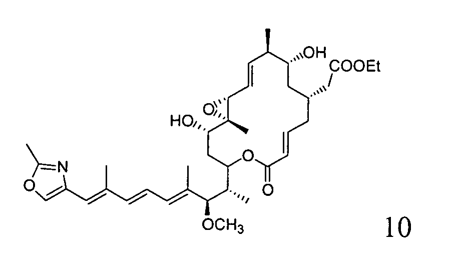

3. Method for producing rhizoxin (1) 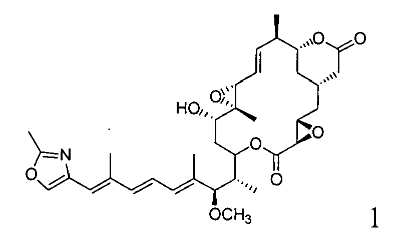 and / or rhizoxin derivatives (2) to (10) 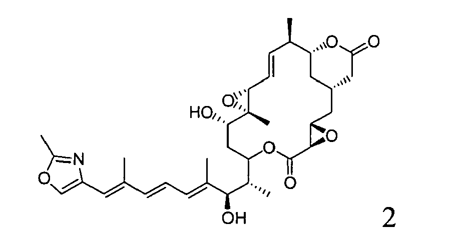 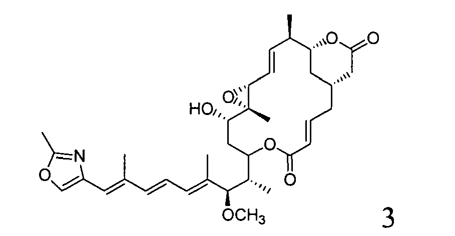 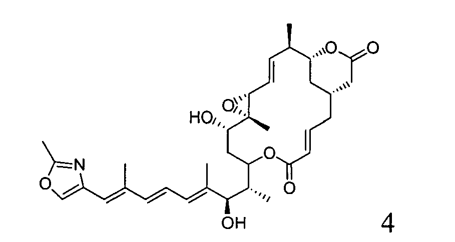 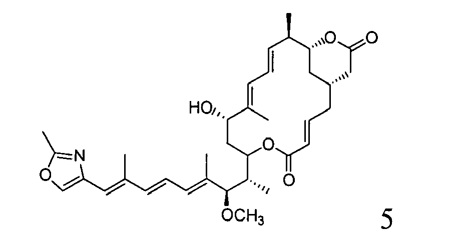 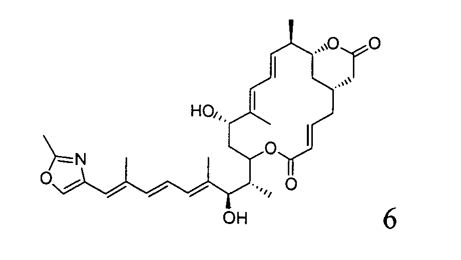 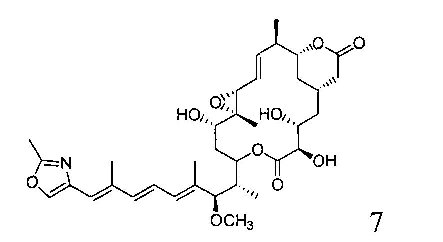 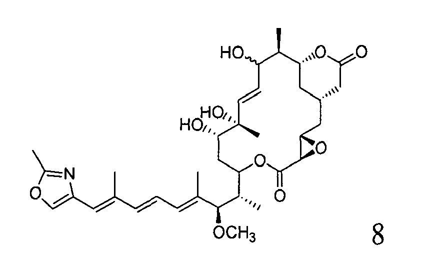 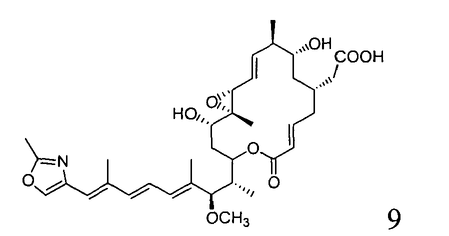 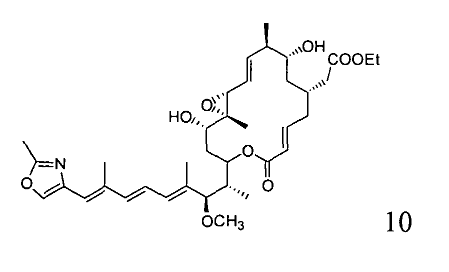 in which a bacterial symbiont of *rhizopus* sp. is cultivated in form of a *Burkholderia rhizoxina* strain in submerse culture until the Rhizoxin (1) and / or rhizoxin derivatives (2) to (10) accumulate in the culture supernatant and are isolated from the culture supernatant. Table 1 is part of this claim.

4. Method for producing rhizoxin (1) and / or rhizoxin derivatives (2) to (10) according to claim 3, wherein the *Burkholderia rhizoxina* strain is DSM 17360.

5. Method for producing rhizoxin (1) and / or rhizoxin derivatives (2) to (10) according to claim 3, wherein the medium contains 1 % cornstarch, 0.5% glycerol, 1% gluten flour, 1% yeast, 1% cornsteep liquour, and 1% CaCO₃.

6. Method for producing rhizoxin (1) and / or rhizoxin derivatives (2) to (10) according to claim 3, wherein the medium is a malt extract peptone medium.

7. Method for producing rhizoxin (1) and / or rhizoxin derivatives (2) to (10) according to claim 5 or 6, wherein the pH-value is 6.5.

8. Method for producing rhizoxin (1) and / or rhizoxin derivatives (2) to (10) according to claim 5 or 6, wherein the fermentation temperature is 30 °C.

9. Method for producing rhizoxin (1) and / or rhizoxin derivatives (2) to (10) according to claim 5 or 6, wherein the cultivation is carried out at 120-200 rpm.

10. Method for producing rhizoxin (1) and / or rhizoxin derivatives (2) to (10) according to claim 4, wherein rhizoxin (1) and / or its derivatives (2) to (10) are isolated from the culture supernatant by extraction with organic solvents or adsorption resins.

## Revendications

1. *Burkholderia rhizoxina* DSM 17360.

2. Utilisation de *Burkholderia rhizoxina* suivant la revendication 1 pour la production de rhizoxine (1) 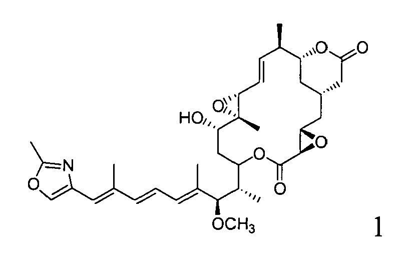 et / ou de dérivés de rhizoxine (2) à (10)

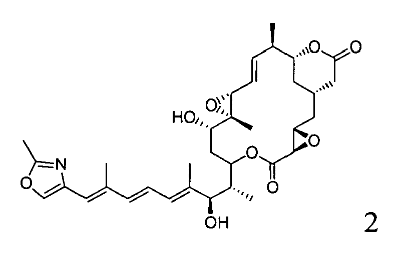

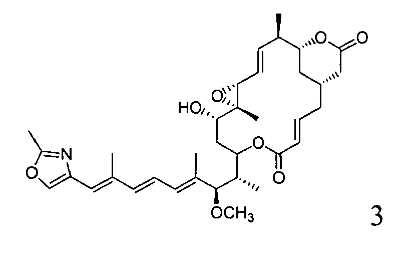

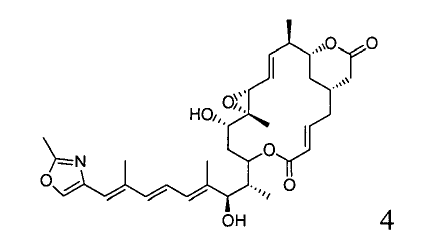

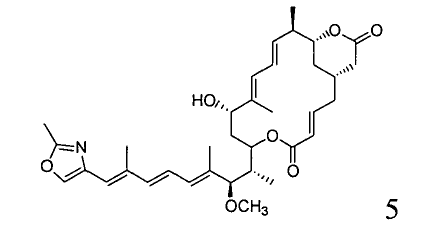

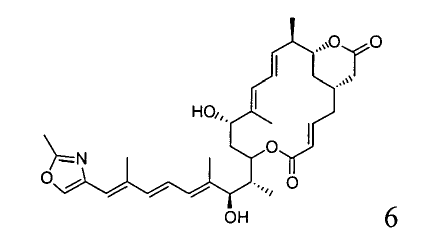

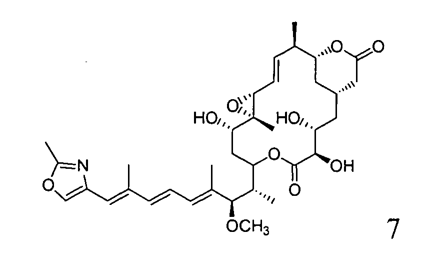

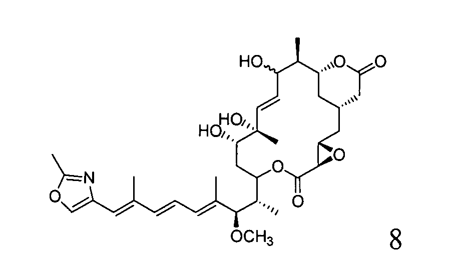

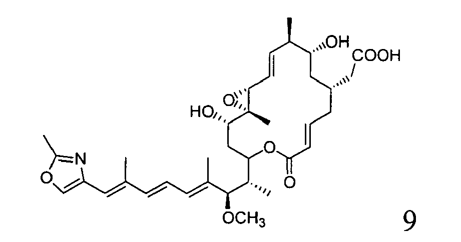

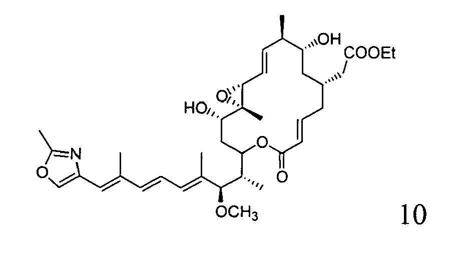

3. Procédé pour la production de rhizoxine (1) 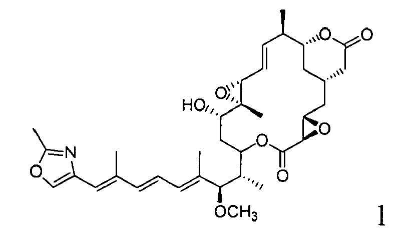 et / ou de dérivés de rhizoxine (2) à (10) 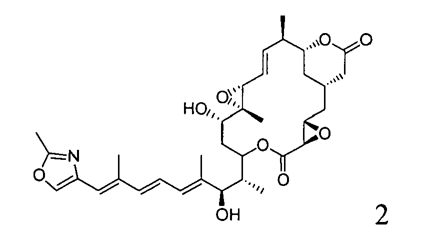 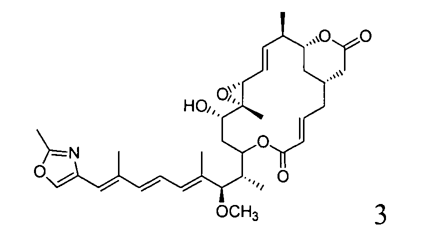 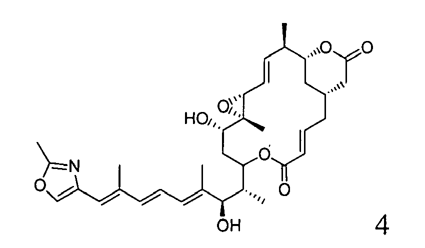 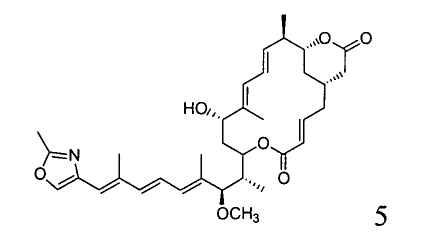 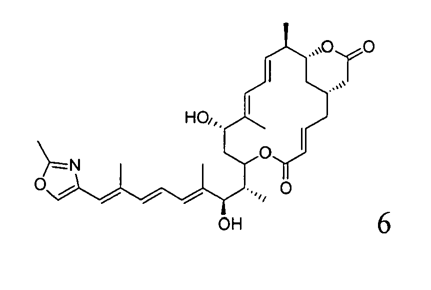 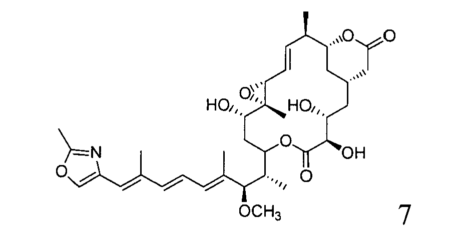 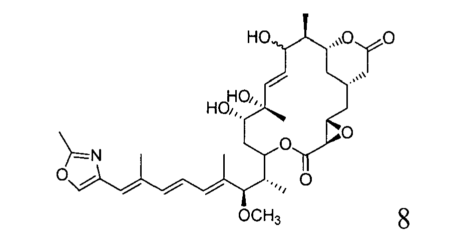 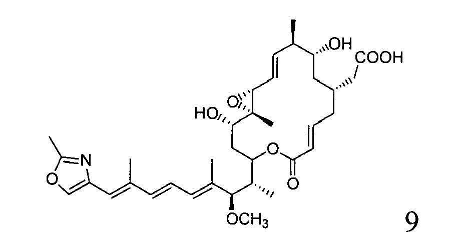 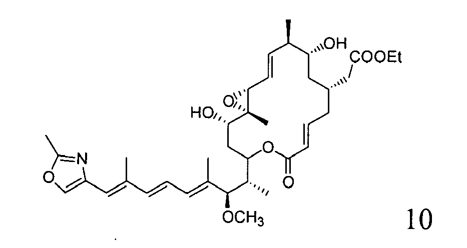 au cours duquel une symbiose bactérienne de *Rhizopus* sp. est cultivée dans les cultures en suspension sous forme d'une souche de *Burkholderia Rhizoxina,* jusqu'à ce que la rhizoxine (1) et / ou les dérivés de rhizoxine (2) à (10) s'accumulent dans les surnageants de culture et soient isolés des surnageants de culture.

4. Le procédé pour la production de rhizoxine (1) et / ou des dérivés de rhizoxine (2) à (10) suivant la revendication 3 est **caractérisé en ce que** la souche de *Burkholderia Rhizoxina* est DSM 17360.

5. Le procédé pour la production de rhizoxine (1) et / ou des dérivés de rhizoxine (2) à (10) suivant la revendication 3 est **caractérisé en ce que** le milieu contient 1 % de amidon de maïs, 0,5 % de glycérole, 1 % de farine de gluten, 1 % de levure, 1 % de Corn Steep et 1 % de CaCO₃.

6. Le procédé pour la production de rhizoxine (1) et / ou des dérivés de rhizoxine (2) à (10) suivant la revendication 3 est **caractérisé en ce que** le milieu est un milieu d'extrait de malt et peptone.

7. Le procédé pour la production de rhizoxine (1) et / ou des dérivés de rhizoxine (2) à (10) suivant les revendications 5 ou 6 est **caractérisé en ce que** la valeur du pH est de 6,5.

8. Le procédé pour la production de rhizoxine (1) et / ou des dérivés de rhizoxine (2) à (10) suivant les revendications 5 ou 6 est **caractérisé en ce que** la température de fermentation est de 30°C.

9. Le procédé pour la production de rhizoxine (1) et / ou des dérivés de rhizoxine (2) à (10) suivant les revendications 5 ou 6 est **caractérisé en ce que** la cultivation est effectuée à 120 - 200 tr/min.

10. Le procédé pour la production de rhizoxine (1) et / ou des dérivés de rhizoxine (2) à (10) suivant la revendication 4 est **caractérisé en ce que** la rhizoxine (1) et / ou ses dérivés (2) à (10) sont isolés des surnageants de culture par extraction avec des solvants organiques ou des résines adsorbantes.
